# EUROPEAN PATENT APPLICATION

(11) **EP 2 255 849 A2**
(43) Date of publication of application: **01.12.2010**
(21) Application number: 10251005.4
(22) Date of filing: 28.05.2010
(51) Int. Cl.: A61Q 17/04, A61Q 19/00, A61K 8/06, A61K 8/73, A61K 8/41

(54) **Topical skin care compositions comprising starch and quaternary ammonium salt**

(30) Priority: 25.03.2010 US 731193; 29.05.2009 US 182284 P
(71) Applicant: Johnson & Johnson Consumer Companies, Inc., Skillman NJ 08558-9418 (US)
(72) Inventor: Candolives, Marilyne, Princeton, NJ 08540 (US); Nystrand, Glenn A., Lebanon, NJ 08833 (US); Walsh, Star M., Upper Black Eddy, PA 18972 (US)
(74) Representative: Kirsch, Susan Edith

(57) **Abstract**

The present invention relates to a composition in the form of a phase-stable oil-in-water emulsion useful for topical skin care products, where the composition includes a quaternary ammonium salt, from about 0.5 to about 4.5 percent by weight of a starch; and at least 20 percent by weight of a hydrophobic agent, where the concentration of the quaternary ammonium salt plus the starch is from about 1.75 to about 5.5 weight percent, based on total weight of the composition.

## Description

### FIELD OF THE INVENTION

The present invention relates to topical skin care compositions in the form of phase-stable oil-in-water emulsions having improved rub-in properties.

### BACKGROUND OF THE INVENTION

The use of emulsions as a product form to topically deliver various ingredients to the skin is well known. Oil-in water emulsions, in particular, are known to provide a non-greasy, aesthetic, skin feel, while still being suitable for delivering relatively low levels of oils or other hydrophobic agents.

The inventors have discovered that it is exceedingly difficult to create a phase-stable emulsion composition that includes a high level of hydrophobic agents, in particular one having a high level of organic sunscreens, without creating undesirable rub-in properties. Undesirable rub-in can manifest itself as the emulsion taking a long period of time to be absorbed by the skin, as well as a visible "whitening" or "soaping" affect that persists during the rub-in period.

Linear silicone (polydimethyl siloxane) fluids have been suggested for use in cosmetic products in order to reduce this unpleasant whitening effect. However, silicone fluids are expensive and not always easy to formulate. As such, the inventors have further recognized that it would be desirable to have an emulsion with high levels of oils or other hydrophobic agents, having good rub-in properties, and including reduced or no levels of silicones.

### SUMMARY OF THE INVENTION

The present invention is directed to a composition comprising a phase-stable oil-in-water emulsion, where the emulsion comprises a continuous aqueous phase and a discontinuous oil phase substantially homogenously suspended in the continuous aqueous phase to form the phase-stable oil-in-water emulsion, the composition comprising from about 0.5 to 4.5 percent by weight of a starch, based on total weight of the composition, a quaternary ammonium salt defined by the Structure I 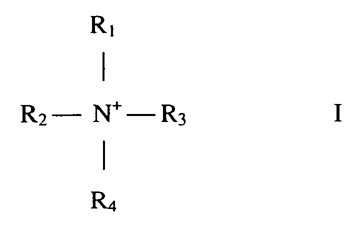 wherein at least one of R₁, R₂, R₃, R₄ comprises a hydrophobic moiety; and 20% or more by weight of a hydrophobic agent, based on total weight of the composition, wherein the concentration of the quaternary ammonium salt plus the starch is from about 1.75 to about 6 percent by weight, based on total weight of the composition.

### DETAILED DESCRIPTION OF THE INVENTION

The present invention meets the aforementioned need and overcomes the disadvantages of the prior art. In particular, it has been discovered that a quaternary ammonium salt can be combined with a starch in a composition having at least 20% by weight or more of hydrophobic agents to form an oil-in-water emulsion composition that is phase-stable and that has reduced levels of undesirable whitening when applied to and rubbed into the skin. As used herein, "phase-stable" means that the continuous aqueous phase and the discontinuous oil phase do not separate into two or more distinct layers composed of hydrophilic components and lipophilic constituents when allowed to remain in a container at 25°C for two weeks.

All percentages included herein are percentages by weight, based on total weight of the composition. Furthermore, as used herein, "substantially free" means the composition contains less than about 1, such as less than about 0.1, e.g., less than about 0.01 weight percent of an ingredient.

Any of a variety of suitable quaternary ammonium salts defined by Structure I may be used in the composition of the present invention. By "quaternary ammonium salt," it is meant an amine having a quaternized nitrogen that has been substituted with at least one hydrophobic moiety.

As used herein, "quaternized nitrogen" means a charged polyatomic cation as represented in Structure I. In general, quaternary ammonium salts have a permanent, pH-independent charge. One skilled in the art will recognize that this pH-independent charge behavior is in contrast to primary ammonium cations (RNH3⁺), secondary ammonium cations (R₂NH2⁺) and tertiary ammonium cations (R₃NH⁺).

In quaternary ammonium salts useful in the present invention, at least one, and desirably at least two, of R₁, R₂, R₃, and R₄ are or include a hydrophobic moiety. In other words, in certain embodiments, the quaternary ammonium salt may include two distinct hydrophobic moieties, each of the two distinct hydrophobic moieties being separately pending from a quaternized nitrogen.

The remainder of the R₁, R₂, R₃ and R₄ groups are, in certain embodiments, also "substituted", i.e. they do not each consist only of a hydrogen atom, but the substitution does not necessarily include a hydrophobic moiety. As such, the remainder of the R₁, R₂, R₃ and R₄ groups may be selected from the group consisting of, for example, methyl, ethyl, propyl and hydroxyl groups. In one embodiment, the remainder of the R₁, R₂, R₃ and R₄ groups are methyl groups.

In a particular embodiment, two of the R₁, R₂, R₃ and R₄ groups include hydrophobic moieties. For example, R₂ and R₃ may each be hydrophobic moieties. In one embodiment, the hydrophobic moieties in R₂ and R₃ are identical. In yet another embodiment, R₂ and R₃ have different carbon chain lengths. For example, R₂ may have a carbon chain length different from R₃ and that is at least 4, such as at least 6, such as at least 8 carbon atoms. In another embodiment, three or four of the R₁, R₂, R₃ and R₄ groups include hydrophobic moieties.

The term "hydrophobic moiety", as used in this specification to describe the above R groups on the quarternary ammonium salt, means any nonpolar, generally water-insoluble group containing four or more carbon atoms. Certain hydrophobic moieties include moieties comprising eight or more carbon atoms, such as 10 or more carbon atoms, e.g., at least 12 carbon atoms, such as 14 or more carbon atoms. The carbon atoms may be arranged in an uninterrupted fashion, e.g., each of the carbon atoms other than those terminating the particular group of carbon atoms has neighboring carbon atoms. Nonlimiting examples of hydrophobic groups include any alkyl, aryl or arylalkyl group, e.g., saturated or unsaturated linear, branched, cyclic, or aromatic hydrocarbon species. Functionalities that may be included in the hydrophobic group may be selected from the group consisting of, for example, ether, ester, ketone, urethane, carbamate and xanthate functionalities.

In one embodiment, the hydrophobic moiety includes an alkyl group having seven or more carbon atoms, such as 10 or more carbon atoms, e.g., 12 or more carbon atoms, such as 14 or more carbon atoms. To provide sufficient hydrophilic character, in certain embodiments of the invention, the quaternary ammonium salt has a hydrophile-lipophile balance that is about 9 or greater, such as about 10 or greater.

In one embodiment, the quaternary ammonium salt is a dialkyl ammonium compound, such as a dialkyl ammonium chloride, such as distearyl dimethylammonium chloride, sold under the trade name VARISOFT TA-100, commercially available from EVONIK Goldschmidt/Degussa GmbH of Essen, Germany. Other suitable quaternary ammonium compounds include "diester quats" that have two hydrophobic moieties, each of which include an alkyl group (tallow, palm oil, or rapeseed oil-derived) and an ester functional group. Another suitable quaternary ammonium salt is distearoylethyl dimonium chloride, sold under the trade name VARISOFT EQ65, also commercially available from EVONIK GmbH. Other examples are sold under the trade name STEPANTEX, e.g., STEPANTEX VK-90. Another suitable quaternary ammonium compound is a "diamidoamine quaternary" sold under the trade name ACCOSOFT. Another suitable quaternary ammonium compound is a "dialkyldimethyl ammonium chloride" sold under the trade name BTC, e.g., BTC 2125M. Yet another suitable quaternary ammonium compound is an arylalkyldiemthyl ammonium chloride, such as an alkylbenzyldimethylammonium chloride, sold under the trade name STEPANQUAT. STEPANTEX, ACCOSOFT, BTC and STEPANQUAT products are available from Stepan Company of Northfield, Illinois. The total concentration of quaternary ammonium salt in the composition may be from about 1% to about 5% by weight of the composition, e.g., from about 1% to about 3.5% by weight. One or more than one quaternary ammonium salt may be used.

One or more starches are also used in the composition. The starch may be of any variety of plant-derived polysaccharides having glucose repeat units joined by alpha acetal linkages to form amylose and/or amylopectin chains. Examples of suitable starches include those derived from such plants as corn, wheat, rice, tapioca, potato, sago, and the like. Such starches can be of a native variety or those developed by plant breeding or by gene manipulation. In an embodiment of the invention, the starches may include either the low amylase variety including less than about 5% amylose, high amylose starches including more than about 40% amylose, and/or combinations thereof. The starch may be of the granular or, so-called, "cooked" (dispersed) variety. Furthermore, the starch may be chemically modified such as by chemical crosslinking via e.g., ionic crosslinking (calcium, aluminum, or phosphate). Also, the starch may be chemically modified to include hydrophobic moieties, or other non-ionic moieties, e.g., hydroxypropyl. In a notable embodiment, the starch is crosslinked and substituted with one or more non-ionic moieties, such as hydrophobic moieties and/or hydroxypropyl moieties. An example of such crosslinked and substituted starches are hydroxypropyl starch phosphates. In another notable embodiment, the starch is absent of any substitutions of non-ionic moieties.

The starch may be of varying particle size. In certain embodiments, the starch has an average particle size that is from about 1 micron to about 100 microns, such as from about 5 microns to about 50 microns, e.g., from about 10 microns to about 40 microns.

An example of a suitable hydrophobically-modified starch is an aluminum starch octenyl succinate, available under the trade name DRY-FLO PURE (aluminum crosslinked, granular). Another suitable example of a non-ionic starch that is substituted with hydroxypropyl groups is a hydroxypropyl starch phosphate, available under the trade name STRUCTURE XL (phosphate crosslinked, dispersed, 100% amylopectin). Examples of such starches are available under the trade names NAVIANCE TAPIOCA, NAVIANCE INSTANT MAIZE and ZEA MAYS corn starch. DRY-FLO PURE and STRUCTURE XL are NAVIANCE starches commercially available from Akzo Nobel SPG LLC of Bridgewater, New Jersey.

The composition may include from about 0.5 to about 4.5 weight percent starch, such as about 1 to about 4.0 weight percent starch, e.g., about 1 to about 3.5 weight percent starch. It has been discovered that emulsions containing relatively high levels of hydrophobic agents, e.g. greater than 20% by weight, and greater than about 4.5% starch, e.g. about 5% by weight, are not phase-stable. In addition, such compositions containing less than about 0.5% of starch, e.g. about 0.25%, exhibit unacceptable whitening.

The composition includes one or more hydrophobic agents. By hydrophobic agents it is meant a molecule that meets one or more of the following three criteria: (a) has a carbon chain of at least six carbons in which none of the six carbons is a carbonyl carbon or has a hydrophilic moiety bonded directly to it; (b) has two or more alkyl siloxy groups; or (c) has two or more oxypropylene groups in sequence. A hydrophobic moiety may include linear, cyclic, aromatic, saturated or unsaturated groups.

One skilled in the art will recognize that "hydrophobic agents" does not include amphiphilic molecules such as emulsifiers, surfactants and other surface active compounds. Amphilphilic molecules that will be understood to be excluded from hydrophobic agents include those compounds that include both of (a) a hydrophobic moiety defined above and (b) a hydrophilic moiety, such as anionic, cationic, zwitterionic, or nonionic group, that is polar, including sulfate, sulfonate, carboxylate, phosphate, phosphonates; ammonium, including mono-, di-, and trialkylammonium species, pyridinium, imidazolinium, amidinium, poly(ethyleneiminium); ammonioalkylsulfonate, ammonioalkylcarboxylate, amphoacetate; hydroxyl, and poly(ethyleneoxy)sulfonyl). Emulsifiers, surfactants and other surface active compounds are commonly used for emulsification and wetting rather than for film-formation, spreading and the like.

Hydrophobic agents used in the present invention are generally insoluble in water and are spreadable across the skin. Examples of suitable hydrophobic agents include emollients, such as mineral oils/waxes, including petrolatum, vegetable oils (glyceryl esters of fatty acids, triglycerides), waxes and other mixtures of fatty esters. Examples of such emollients include, without limitation, isopropyl palmitate and dicaprylyl ether. Another class of suitable hydrophobic agents for use in the present invention is organic ultraviolet (UV) filters.

Organic UV filters that are useful hydrophobic agents in the present invention are compounds that absorb radiation in the UV range. Examples of organic UV filters include, without limitation, 3-benzylidene camphor, specifically 3-benzylidene norcamphor and derivatives thereof, e.g. 3-(4-methylbenzylidene) camphor; 4-aminobenzoic acid derivatives, specifically 4-(dimethylamino)benzoic acid-2-ethylhexyl esters, 4-(dimethylamino)benzoic acid-2-octyl esters and 4-(dimethylamino)benzoic acid amylesters; esters of cinnamonic acid, in particular 4-methoxycinnamonic acid-2-ethylhexylester, 4-methoxycinnamonicacid propylester, 4-methoxycinnamonic acid isoamyl ester, 2-cyano-3,3-phenylcinnamonic acid-2-ethylhexyl ester (octocrylene); esters of salicylic acid, i.e., salicylic acid-2-ethylhexylester, salicylic acid-4-isopropylbenzyl ester, salicylic acid homomenthyl ester; derivatives of benzophenones, in particular 2-hydroxy-4-methoxybenzophenone, 2-hydroxy-4-methoxy-4'-methylbenzophenone, 2,2'-dihydroxy-4-methoxybenzophenone; esters of benzalmalonic acid, in particular 4-methoxybenzmalonic acid di-2-ethylhexyl ester; triazine derivatives, for example 2,4,6-trianilino-(p-carbo-2'-ethyl-1'-hexyloxy)-1,3,5-triazine and octyltriazone; or benzoic acid, 4,4'-[[6-[[[(1,1- dimethylethyl)amino]carbonyl]phenyl]amino]-1,3,5-triazine-2,4-diyl]diimino]bis-, bis (2-ethylhexyl) ester (UVASORB HEB); propane-1,3-diones, for example, 1-(4-tert.butylphenyl)-3-(4'-methoxyphenyl) propane-1,3-dione; ketotricyclo (5.2.1.0) decane derivatives; derivatives of benzoylmethane, for example, 1-(4'-tert.butylphenyl)-3-(4'-methoxyphenyl)propane-1,3-dione, 4-tert-butyl-4'-methoxydibenzoylmethane (PARSOL 1789), 1-phenyl-3-(4'-isopropylphenyl)-propane-1,3-dione, derivatives of benzoic acid 2-(4-diethylamino-2-hydroxybenzoyl)-benzoic acid hexylester (UVINUL A+), or 1H-benzimidazole-4,6-disulfonic acid, 2,2'-(1,4-phenylene)bis-, disodium salt (NEO HELOPAN AP); and benzotriazoles, in particular the benzotriazole derivative known as 2,2'-methylene-bis-(6-(2H-benzotriazole-2-yl)-4-(1,1,3,3-tetramethylbutyl)-phenol) [INCI: Bisoctyltriazol], which is commercially available under the tradename TINOSORB M from CIBA Chemicals. Another useful benzotriazole derivative is 2-(2H-benzotriazole-2-yl)-4-methyl-6-[2-methyl-3-[1,3,3,3-tetramethyl-1-[(trimethylsilyl)oxy]disiloxanyl]propyl]-phenol (CAS-No.: 155633-54-8), also identified by the INCI name drometrizole trisiloxane and available from Chimex under the tradename MEXORYL XL.

Oil-soluble broadband filters include the asymmetrically substituted triazine derivatives. Of particular interest is 2,4-bis-{[4-(2-ethyl-hexyloxy)-2-hydroxy]-phenyl}-6-(4-methoxyphenyl)-1,3,5-triazine (INCI: anisotriazine) that is commercially available under the tradename TINOSORB S from CIBA Chemicals.

The composition comprises about 20 or more total weight percent of one or more hydrophobic agents, such as from about 20weight percent hydrophobic agents to about 50weight percent hydrophobic agents, e.g., 20weight percent hydrophobic agents to about 40weight percent hydrophobic agents, such as from about 20weight percent hydrophobic agents to about 30weight percent hydrophobic agents.

Compositions of the present invention are oil-in water (O/W) emulsions. By O/W emulsions it is meant that a discontinuous oil phase is substantially homogenously suspended in a continuous aqueous phase. The continuous aqueous phase generally includes water (i.e., is ionically conductive), as well as other ingredients that are soluble, miscible or dispersed with the water. The concentration of water in the composition may be from about 10 weight percent to about 78 weight percent, such as from about 35 weight percent to about 65 weight percent, e.g., from about 40 weight percent to about 60 weight percent. The oil phase includes the hydrophobic agents, as well as other optional ingredients that are soluble, miscible or partitioned in with the hydrophobic agents.

The inventors have found that, in order to provide combined properties of phase stability and reduced undesirable whitening in compositions according to the present invention, the starch and the quaternary ammonium salt should be present in a total combined concentration of starch plus quaternary ammonium salt that is from about 1.75% weight percent to about 6 weight percent, such as from about 2 weight percent to about 6 weight percent, such as from about 2 weight percent to about 5.5 weight percent, e.g., 4 weight percent to about 5.5 weight percent.

Compositions of the present invention are particularly suitable for use in daily-use sunscreen lotions. By daily-use sunscreen lotions, it is meant lotions that include UV-screening agents that may provide SPF protection, such as SPF 15 to about SPF 45, but do not necessarily require water resistance. As such, compositions of the present invention may be substantially free of waterproofing agents that are commonly used in "recreational sunscreens" to maintain SPF protection after exposure to water, such as is typically tested in the commonly used "Colipa" test methods for SPF analysis. Waterproofing agents that may be excluded from the composition include for example those based on PVP and acryclic or methacrylic esters. Examples of waterproofing polymers include PVP/Hexadecene Copolymer (GANEX V-216®), PVP/Eicosene Copolymer (GANEX V-220^{®}), PVP/Ethyl Methacrylate/Methacrylic Acid Copolymer, Ammonium Acrylates Copolymer, and Polyolprepolymer-2. GANEX materials are avilable from ISP of Wayne, NJ. Polyolprepolymer-2 is available as TOPICARE DELIVERY COMPOUNDS from Penederm Inc, 320 Lakeside Dr., Foster City, California (now owned by Mylan Industries of Canonsburg, California).

In another embodiment, the composition comprises an additional emulsifier that does not meet the requirements defined above to be a quaternary ammonium salt. The additional emulsifier is, in certain embodiments, a non-ionic emulsifier, present in a concentration of about 0.1 to about 5.0 weight percent, such as from about 1 to about 3 weight percent. Examples of suitable additional non-ionic emulsifiers include fatty alcohols such as cetearyl alcohol, fatty esters, Poloxamer 184, laureth-4, sorbitan esters such as sorbitan trioleate and sorbitan stearate, polyoxyethylene-(2) oleyl ether, polyoxyethylene ethers of fatty alcohols such as ceteareth-20, isocetheth-20, cetearyl glucoside, glyceryl oleate, trideceth-9, polyethylene glycol-40 hydrogenated castor oil, and mixtures thereof.

In another embodiment, the composition is substantially free of silicones. Silicones, as defined herein, are generally hydrophobic compounds having a silicon atom that is bonded to at least one oxygen atom and at least one carbon atom, e.g., a carbon atom that is a part of a methyl or other alkyl group. The silicones that the composition is substantially free of are, in certain embodiments, liquids at room temperature, but in other embodiments may be solid at room temperature, such as silicone polymers and silicone gums.

In one embodiment, the composition includes a humectant that serves to enhance spreadibility and/or moisture retention. Any of a variety of commercially available humectants, which are capable of providing moisturization and conditioning properties to the personal cleansing composition, is suitable for use in the present invention. Examples of suitable humectants nonexclusively include: 1) water soluble liquid polyols selected from the group comprising glycerine, propylene glycol (e.g., 1-2 or 1-3 propanediol), hexylene glycol, butylene glycol, dipropylene glycol, polyglycerols, and mixtures thereof; 2) polyalkylene glycols of the formula: HO-(R"O)_{b}-H, wherein R" is an alkylene group having from about 2 to about 3 carbon atoms and b is an integer of from about 2 to about 10; 3) polyethylene glycol ether of methyl glucose of formula CH₃-C₆H₁₀O₅-(OCH₂CH₂)_{c}-OH, wherein c is an integer from about 5 to about 25; 4) urea; f) grain flours such as oat flour that has been milled to a particle size of about 100 microns and is suitable for forming colloidal dispersions in the inventive composition, and 5) mixtures thereof. In one embodiment, the humectant is a polyhydric alcohol such as glycerol or a propanediol such as propylene glycol. The humectant may be present in an amount of from about 1% to about 70% by weight in composition, such as from about 1% to about 40% by weight, e.g., from about 5% to about 30%, such as from about 1% to about 25%, based on the overall weight of the composition.

Compositions of the present invention may include a film-forming polymer in order to aid in film formation on the skin. Examples of film-forming agents include, but are not limited to or proteins and synthetic polymers such as polyesters, polyacrylics, polyurethanes, vinyl polymers, polysulfonates, polyureas, polyoxazolines, and the like. Specific examples of film-forming polymers include, for example, hydrogenated dimer dilinoleyl/dimethylcarbonate copolymer, available from Cognis Corporation of Ambler, Pennsylvania under the trade name COSMEDIA DC; water-dispersible polyesters, including sulfopolyesters such those commercially available from Eastman Chemical as EASTMAN AQ 38S. The amount of film-forming polymer present in the composition may be from about 0.1% to about 5%, or from about 0.1% to about 3%, or from about 0.1% to about 2%.

Compositions of the present invention may include a skin benefit agent. A benefit agent is any element, an ion, a compound (e.g., a synthetic compound or a compound isolated from a natural source) or other chemical moiety in solid (e.g. particulate), liquid, or gaseous state and compound that has a cosmetic or therapeutic effect on the skin, hair, mucosa, or teeth. As used herein, the term "benefit agent" includes any active ingredient such as a cosmetic or pharmaceutical, that is to be delivered into and/or onto the skin, hair, mucosa, or teeth at a desired location.

The benefit agents useful herein may be categorized by their therapeutic benefit or their postulated mode of action. However, it is to be understood that the benefit agents useful herein may, in some circumstances, provide more than one therapeutic benefit or operate via greater than one mode of action. Therefore, the particular classifications provided herein are made for the sake of convenience and are not intended to limit the benefit agents to the particular application(s) listed.

Examples of suitable benefit agents include those that provide benefits such as, but not limited to, depigmentation agents; amino acids and their derivatives; anti-acne agents; anti-aging agents; anti-wrinkling agents; shine-control agents; antipruritic agents; hair growth inhibitor agents; anti-infective agents; anti-inflammatory agents; wound healing promoters; peptides, polypeptides and proteins; medicament agents; skin firming agents; vitamins; skin lightening agents; skin darkening agents; depilating agents; counterirritants; insecticides; poison ivy products; anti-diaper rash agents; prickly heat agents; herbal extracts; vitamin A and its derivatives; flavonoids; sensates and stress-reducing agents; anti-oxidants; keratolytics; inorganic pigments; and thickening agents.

The amount of the benefit agent that may be used may vary depending upon, for example, the ability of the benefit agent to penetrate through the skin, nail, mucosa, or teeth; the specific benefit agent chosen, the particular benefit desired, the sensitivity of the user to the benefit agent, the health condition, age, and skin and/or nail condition of the user, and the like. In sum, the benefit agent is used in a "safe and effective amount," which is an amount that is high enough to deliver a desired skin or nail benefit or to modify a certain condition to be treated, but is low enough to avoid serious side effects, at a reasonable risk to benefit ratio within the scope of sound medical judgment.

The pH of the present compositions is not critical, and is typically from about 3.5 to about 8, for example, from about 4 to about 7, e.g., from about 4 to about 6.

The composition is topically applied by means of directly laying on or spreading on outer skin, e.g., by use of the hands or an applicator such as a wipe, roller, or spray.

Compositions of the present invention are typically extrudable or dispensable from a package, such as may be caused to flow from the package to be applied directly or indirectly, topically to the body or another surface. Depending upon the particular function, compositions of present invention are desirably rubbed onto the skin and allowed to remain without rinsing.

Particularly suitable uses for compositions of the present invention include application onto human skin, such as for applications in which the composition is allowed to remain on the skin for at least an hour. Such "leave-on" applications for the inventive compositions include skin lotions such as sunscreens, especially daily use sunscreen lotions, skin moisturizers and skin conditioners, among other personal care applications.

As discussed above, applicants have discovered unexpectedly that compositions of the present invention provide personal care products having high loading of hydrophobic agents without compromising aesthetics, such as is typified by high levels of skin whitening for comparable prior art compositions. Furthermore, compositions of the present inventions are phase-stable.

The following non-limiting examples further illustrate the claimed invention.

### EXAMPLES

### I. Comparative Examples C1-C3

The following comparative examples were prepared. Comparative Example C1, shown in Table 1, is consistent with prior art.

**TABLE 1: Comparative Example, C 1**

| **Trade Name** | **INCI Name** | **% w/w** |
|---|---|---|
| *Phase A* | | |
| Deioinized Water | Deioinized Water | 70.50 |
| Structure XL | Hydroxypropyl Starch Phosphate | 5.00 |
| Phenonip XB | Phenoxyethanol, Methylparaben, Ethylparaben, Propylparaben | 1.00 |

| *Phase B* | | |
|---|---|---|
| Imwitor 375 | Glyceryl/Citrate/Lactate/Linoleate/Oleate | 3.0 |
| Propal NF | Isopropyl Palmitate | 2.0 |
| Parsol MCX | Octinoxate | 12.0 |
| Parsol 1789 | Avobenzone | 1.5 |
| Vitamin E Acetate | Tocopheryl Acetate | 1.0 |
| Finsolv TN | C₁₂₋₁₅ Alkyl Benzoate | 2.0 |
| Drakeol 35 Light Mineral Oil | Mineral Oil | 2.0 |

STRUCTURE XL, PHENONIP, IMWITOR, PROPAL, and PARSOL are available from Akzo Nobel (Bridgewater, NJ), Nipa/Clariant (Leeds, UK), Sasol (Johannesburg, South Africa), Lubrizol (Wickliffe, Ohio), DSM (Bern, Switzerland), Innospec (Edison, NJ) and Penreco (Karns City, PA) respectively.

Comparative Example C1 was prepared by premixing Phase A ingredients, adding STRUCTURE XL & allowing to mix for 5 minutes, and heating to 75°C-80°C. Phase B was premixed, heated to 75°C-80°C with mixing until all ingredients were dissolved. Phase B was added to Phase A at 75°C-80°C while mixing at 900 rpm. The mixture was allowed to mix for 10 minutes.

Comparative Example C2, shown in Table 2, is similar to Comparative Example 1, but has a higher concentration of hydrophobic agents, 25.7%, rather than 18.5%. Note that the sunscreens, vitamin E, FINSOLV and mineral oil are considered hydrophobic agents, for purposes of this disclosure.

**TABLE 2: Comparative Example, C2**

| **Trade Name** | **INCI Name** | **% w/w** |
|---|---|---|
| *Phase A* | | |
| Deioinized Water | Deioinized Water | 64.2 |
| Structure XL | Hydroxypropyl Starch Phosphate Phenoxyethanol, Methylparaben, , | 4.7 |
| Phenonip XB | Ethylparaben, Propylparaben | 0.9 |

| *Phase B* | | |
|---|---|---|
| Imwitor 380 | Glyceryl /Citrate/Lactate/Linoleate/Oleate | 2.8 |
| Propal NF | Isopropyl Palmitate | 1.9 |
| Parsol MCX | Octinoxate | 18.4 |
| Parsol 1789 | Avobenzone | 2.4 |
| Vitamin E Acetate | Tocopheryl Acetate | 0.9 |
| Finsolv TN | C₁₂₋₁₅ Alkyl Benzoate | 1.9 |
| Drakeol 35 Light | | |
| Mineral Oil | Mineral Oil | 1.9 |

Comparative Example C2 was prepared in a similar manner to Comparative Example C1. Comparative Example C2, however, was not phase-stable in that it phase-separated after two weeks at 25°C.

Comparative Example C3, shown in Table 3, is nearly identical to Comparative Example 2, except that the concentration of starch was also 7.2%. Comparative Example C3 was also prepared in a similar manner to Comparative Example C1.

**TABLE 3: Comparative Example, C3**

| **Trade Name** | **(INCI Name** | **% w/w** |
|---|---|---|
| *Phase A* | | |
| Deioinized Water | Deioinized Water | 63.1 |
| Structure XL | Hydroxypropyl Starch Phosphate | 7.2 |
| | Phenoxyethanol, Methylparaben, Ethylparaben, | |
| Phenonip XB | Propylparaben | 0.9 |

| *Phase B* | | |
|---|---|---|
| Imwitor 380 | Glyceryl /Citrate/Lactate/Linoleate/Oleate | 2.7 |
| Propal NF | Isopropyl Palmitate | 1.8 |
| Parsol MCX | Octinoxate | 17.6 |
| Parsol 1789 | Avobenzone | 2.2 |
| Vitamin E Acetate | Tocopheryl Acetate | 0.9 |
| Finsolv TN | C₁₂₋₁₅ Alkyl Benzoate | 1.8 |
| Drakeol 35 Light | | |
| Mineral Oil | Mineral Oil | 1.8 |

Comparative Example C3 was also not phase-stable. The results of these experiments suggest that such prior art compositions are not phase-stable.

### II. Inventive Example E1 and Comparative Example C4

The inventive composition, E1, and Comparative Example, C4, are shown in Table 4 below.

**TABLE 4:**

| | | **Inventive Example E1** | **Comparative Example C4** |
|---|---|---|---|
| **Ingredient Trade Name** | **CTFA Name** | **% (w/w)** | **% (w/w)** |
| Deionized Water | Water | 46.69 | 46.69 |
| Versene NA | Disodium EDTA | 0.10 | 0.10 |
| Glycerin | Glycerin | 6.00 | 6.00 |
| Methylparaben | Methylparaben | 0.00 | 0.25 |
| Crodacol CS 50 | Cetearyl Alcohol | 1.50 | 1.50 |
| Cetiol CC | Dicaprylyl Carbonate | 4.00 | 4.00 |
| Corapan TQ | Diethylhexyl, 2-6 Naphthalate | 0.10 | 0.00 |
| Neoheliopan OS | Ethylhexyl salicylate | 5.00 | 5.00 |
| Uvinul 539T | Octocrylene | 3.55 | 3.55 |
| Parsol 1789 | Avobenzone | 3.06 | 3.06 |
| Neoheliopan BB | Benzophenone-3 | 5.00 | 5.00 |
| Neohelipan HMS | Homosalate | 6.00 | 6.00 |
| Montanov L | C₁₄₋₂₂ Alcohols & C₁₂₋₂₀ Alkyl Glucoside | 1.00 | 1.00 |
| Varisoft EQ65 | Distearoylethyl Dimonium Chloride (and) Cetearyl Alcohol | 3.50 | 3.50 |
| BZK 50% Active | Benzalkonium Chloride | 0.00 | 0.10 |
| Dermasoft Octiol H | Caprylyl Glycol & Hinokitiol | 0.50 | 0.00 |
| Deionized Water | Water | 13.00 | 13.00 |
| Colloidal Oat Flour- | Oat (Avena Sativa) Flour | 1.00 | 1.00 |
| Gamma Treated | | | |
| Structure XL | Hydroypropyl Starch Phosphate | 1.50 | 0.00 |
| Spheron L-1500 | Silica | 0.00 | 0.25 |

Inventive Example E1 was prepared using the following process:
E1 was prepared by premixing water-soluble ingredients and heating to 75°C-80°C until all ingredients were dissolved. Oil-soluble ingredients were premixed and heated to 75°C-80°C with mixing until all ingredients were melted. The oil phase was added to the water phase at 75°-80°C while mixing at 900 rpm. The mixture was allowed to mix for 10 minutes, then homogenized at 6000 rpm for 1 minute. The batch was then left mixing and cooling and the preservative Structure XL was added below 40°C.

C4 was made using a similar process, but had no hydroxypropyl starch phosphate. This formula also had slight differences in preservative, silica and napthalate level, but was otherwise identical to E1. E1 and C4 were compared for undesirable whitening on the skin by 20 panelists. The panelists were asked to choose which of the two compositions showed more whitening, or alternatively, indicate there was no discernible difference. Fifteen of the twenty panelists indicated Comparative Example C4 showed more undesirable whitening, while three of the twenty panelists indicated E 1 showed more undesirable whitening, and two panelists found no discernible difference. It is reasonable to conclude from this experiment that the addition of starch, at levels according to the present invention, to a composition containing the quaternary ammonium salt, and having relatively high loading of hydrophobic agent, reduces undesirable whitening, while maintaining phase stability.

**III. Inventive Examples E2-E8**
The following inventive examples including over 22% of organic sunscreen hydrophobic agents were prepared. Inventive Example E2 is shown in Table 5.

**TABLE 5**

| | | | |
|---|---|---|---|
| | **INCI Name** | **Trade Name** | **% w/w** |
| Main Phase | Purified Water | Purified Water | 62.30 |
| | Disodium Edetate | Disodium EDTA | 0.10 |
| | Panthenol | D-Panthenol | 0.10 |
| | Glycerin | Glycerin | 1.00 |
| Oil Phase | C₁₂₋₁₅ Alkyl Benzoate | Finsolv TN | 4.00 |
| | Ethylhexyl Salicylate | Neo Heliopan OS | 5.00 |
| | Oxybenzone | Neo Heliopan BB | 5.00 |
| | Stearyl Alcohol | Stearyl Alcohol | 1.00 |
| | Varisoft TA-100 | Distearyldimonium Chloride | 1.00 |
| | Ceteareth-20 | Promulgen-D | 3.00 |
| | Flaxseed Oil | Para Oil Flaxseed Oil | 0.20 |
| | Octocrylene | Neo Heliopan 303 | 3.55 |
| | Homosalate | Neoheliopan HMS | 6.00 |
| | Avobenzone | Parsol 1789 | 3.00 |
| | Neolone Cap G | Caprylyl Glycol & MIT | 0.85 |
| | Aciphyte of Rosemary GL | Rosmarinus Officinalis (Rosemary) Leaf Extract n (and) Glycerin | 0.10 |
| | Fragrance | Fragrance Oil Z9573 safety Mod UR226 | 0.20 |
| | Tocopheryl Acetate | dl-Alpha Tocopheryl Acetate | 0.10 |
| | Hydroxypropyl Starch Phosphate | Structure XL | 3.50 |

Inventive Example E2 was prepared by premixing Phase A ingredients and heating. EDTA was added when the temperature reached 38°C-42°C. Glycerin and panthenol were added while continuing to mix and the mixture was heated to 70°C-75°C.

Phase B ingredients were premixed and heated to 75°C-80°C with mixing until all ingredients were dissolved. Phase B was added to Phase A at 75°C-80°C while mixing. The mixture was allowed to cool. At 45°C, the NEOLENE CAP G was added. The emulsion was cooled to 40°C and fragrance, rosemary, and tocopherol acetate were added. After mixing for 1 minute, STUCTURE XL was added.

Inventive Examples E3-E8 were identical to Inventive Example E2, except that different starches were used, as shown in Table 6. For Inventive Examples E3, E5, E7 and E8, the starch was added at the end of the process, as was for Example E2. For Inventive Examples E4 and E6, the starch was added in the water phase. For Inventive Example E4, the starch was dispersed in cold water, heated for 30 minutes at 70°C-80°C.

**TABLE 6**

| **Example** | **Trade Name** | **INCI Name** | **Function** | **Source** | **Type** | **Undesirable Whitening (Scale 0-10; 10= highest whitening)** |
|---|---|---|---|---|---|---|
| E2 | Structure XL | Hydroxypropyl Starch Phosphate | rheology modifier / aesthetic modifier | Corn | Dispersed | 0.5 |
| E3 | Naviance Instant Maize | Zea Mays Corn Starch | rheology modifier | Corn | Dispersed | 3 |
| E4 | Naviance Tapioca | Tapioca Starch | rheology modifier/film former | Tapioca | Granular | 3 |
| E5 | Naviance Tapioca P | Tapioca Starch | aesthetic modifier | Tapioca | Granular | 1.5 |
| E6 | Structure Solonace | Potato Starch Modified | low pH rheology modifier/ emulsion stabilizer | Potato | Granular | 4 |
| E7 | Zea Mays Corn Starch | Corn starch | aesthetic modifier | Corn | Granular | 1.5-2 |
| E8 | Dry Flo Pure | Aluminum Starch Octenyl Succinate | aesthetic modifier | Corn | Granular | 2 |

Also shown in Table 6 is the amount of whitening of the particular composition. In this particular experiment, the magnitude of whitening was determined by the following method. A positive displacement pipette was used to dispense 0.1 mL of test composition onto the center of a 2 inch x 3 inch area of the forearm. The first two sections of the index and middle fingers were used to move the product at a rate of 1 rotation per second in an outward motion. The whiteness of the product was evaluated on the 5^{th} rotation by two panelists having significant training in sensory evaluation of skin care products. Each test composition was rated from 0 (no whitening) to 10 (complete whitening).

The magnitude of whitening, i.e. the whitening score, is reported in Table 6. Increase in whitening scores indicates an increase in whitening. Whitening scores up to and including 5 are considered acceptable. The results suggest that, while variation exists in the amount of whitening, starches of various sources and types are suitable for use in the present invention. DRY-FLO PURE is a granular, non-ionic, aluminum crosslinked, granular starch having hydrophobic (octenyl succinate) substitution. TAPIOCA, NAVIANCE INSTANT MAIZE and ZEA MAYS are granular starches that are not substituted. Particularly effective at reducing whitening is STRUCTURE XL, a dispersed, phosphate-crosslinked, non-ionic, hydroxypropylated starch having 100% amylopectin units. NAVIANCE DRY-FLO PURE and STRUCTURE XL are NAVIANCE starches and are commercially available from Akzo Nobel SPG LLC of Bridgewater, New Jersey.

### IV. Inventive Example E9

Inventive Example E9 was prepared substantially as Inventive Example E2, except that the five sunscreens (Neo-Heliopan OS, BB, 303, HMS; and PARSOL 1789) were replaced with a blend of emollients, specifically isopropyl palmitate, mineral oil and dicaprylyl ether, in equal proportions. The phase-stable composition was tested for whitening and was determined to have a score of 2. This suggests that the hydrophobic agent may be selected from a range of suitable compounds.

### V. Inventive Examples E9-E13 and Comparative Examples C5-C8

A composition having 1% quaternary ammonium salt, as with Inventive Example E2, was prepared, except that the concentration of starch was varied, as shown in Table 7, below.

**TABLE 7**

| **Example** | **% Starch** | **Whitening 0-10 (10= highest whitening)** |
|---|---|---|
| C5 | 8% Structure XL | formula separated |
| C6 | 5% Structure XL | formula separated |
| E10 | 4.5% Structure XL | 0.25 |
| E11 | 4% Structure XL | 0.5 |
| E12 | 1.5% Structure XL | 2 |
| E13 | 1% Structure XL | 3.5 |
| C7 | 0.5% Structure XL | 5.5-6 |
| C8 | 0.25% Structure XL | 6 |

Comparative Examples C5 and C6, with relatively high levels of starch, were not phase-stable. Comparative Example C7 and C8, with relatively low levels of starch, had unacceptably high whitening. Inventive Examples E10-E13, with intermediate levels of starch, had relatively low whitening and were phase-stable. This suggests that compositions according to the present invention are suitable to provide both phase stability and acceptable whitening.

### VI. Inventive Examples E14-E17 and Comparative Examples C9-C10

Compositions substantially identical to Inventive Example E1 were prepared, except that the concentration of quaternary ammonium salt was varied, as shown in Table 8, below.

**TABLE 8**

| **Example** | **% Quaternary Ammonium Salt** | **Whitening Scale 0-10 (10= highest whitening)** |
|---|---|---|
| C9 | 3.5% Varisoft EQ65, 4.5% Structure XL | Separated |
| C10 | 3.5% Varisoft EQ65, 3.5% Structure XL | Separated |
| E14 | 3.5% Varisoft EQ65, 2.5% Structure XL | 0.5 |
| E15 | 3.5% Varisoft EQ65, 1.5% Structure XL | 1-1.5 |
| E16 | 3.5% Varisoft EQ65, 1% Structure XL | 2.5 |
| E17 | 3.5% Varisoft EQ65, 0.5% Structure XL | 4 |

Comparative Example C9 and C10, as observed, were not phase-stable, while Inventive Examples E14-E17 had relatively low whitening and were phase-stable. This again shows that compositions according to the present invention provide both phase stability and acceptable whitening.

## Claims

1. A composition comprising a phase-stable, oil-in-water emulsion, said emulsion comprising a continuous aqueous phase and a discontinuous oil phase substantially homogenously suspended in the continuous aqueous, said composition comprising:
from about 0.5 to about 4.5 percent by weight of a starch, based on total weight of the composition,
a quaternary ammonium salt defined by the Structure I: 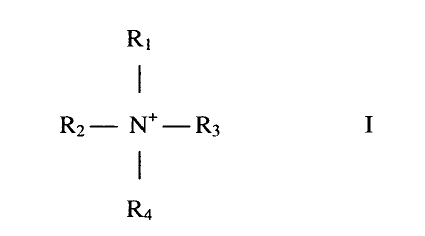 wherein at least one of R₁, R₂, R₃, R₄ comprises a hydrophobic moiety; and 20 or more percent by weight of a hydrophobic agent, based on total weight of the composition,
wherein the concentration of the quaternary ammonium salt plus the starch is from about 1.75 to about 6 percent by weight, based on total weight of the composition.

2. The composition of claim 1, wherein at least two of R₁, R₂, R₃, and R₄ comprise a hydrophobic moiety.

3. The composition of claim 1 or claim 2, wherein the hydrophobic moieties each comprise 10 or more carbon atoms.

4. The composition of any one of the preceding claims, comprising from about 1 to about 5 percent by weight of the quaternary ammonium salt.

5. The composition of any one of the preceding claims, wherein the composition comprises from about 1 to about 4 percent by weight of the starch.

6. The composition of any one of the preceding claims, wherein the concentration of the quaternary ammonium salt plus the starch is from about 2 to about 5.5 percent by weight.

7. The composition of any one of the preceding claims, further comprising a non-ionic emulsifier.

8. The composition of any one of the preceding claims, which is substantially free of silicones.

9. The composition of any one of the preceding claims, wherein the hydrophobic agent comprises an organic UV filter.

10. The composition of any one of the preceding claims, wherein the hydrophobic agent is an organic UV filter.
